(19) Europäisches Patentamt
European Patent Office
Office européen des brevets

(11) **EP 3 122 231 B1**

(12) **EUROPÄISCHE PATENTSCHRIFT**

(45) Veröffentlichungstag und Bekanntmachung des Hinweises auf die Patenterteilung:
**11.03.2020 Patentblatt 2020/11**

(21) Anmeldenummer: **15720586.5**

(22) Anmeldetag: **30.03.2015**

(51) Int Cl.:
*A61B 17/00* (2006.01)    *A61B 17/34* (2006.01)
*A61M 13/00* (2006.01)

(86) Internationale Anmeldenummer:
**PCT/DE2015/000152**

(87) Internationale Veröffentlichungsnummer:
**WO 2015/144120 (01.10.2015 Gazette 2015/39)**

(54) **VORRICHTUNG ZUR REGELUNG DES KÖRPERINNENDRUCKS BEI VERWENDUNG EINER MEDIZINTECHNISCHEN PUMPE**

DEVICE FOR REGULATING THE BODY'S INTERNAL PRESSURE DURING USE OF A MEDICAL PUMP

DISPOSITIF POUR LE RÉGLAGE DE LA PRESSION INTERNE DU CORPS LORS DE L'UTILISATION D'UNE POMPE MÉDICALISÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.03.2014 DE 102014004480**

(43) Veröffentlichungstag der Anmeldung:
**01.02.2017 Patentblatt 2017/05**

(73) Patentinhaber: **W.O.M. World of Medicine GmbH 10587 Berlin (DE)**

(72) Erfinder: **ZEYSSIG, Andreas 10245 Berlin (DE)**

(74) Vertreter: **Jungblut & Seuss Patentanwälte Max-Dohrn-Strasse 10 10589 Berlin (DE)**

(56) Entgegenhaltungen:
**WO-A1-2013/000777    GB-A- 2 486 018**
**US-A1- 2004 039 243    US-A1- 2005 085 799**
**US-A1- 2010 094 113    US-A1- 2012 283 691**
**US-A1- 2014 088 508    US-B1- 6 557 553**
**US-B1- 8 545 416**

**Beschreibung**

Die vorliegende Erfindung betrifft ein Verfahren zur Regelung des Körperinnendrucks, z. B. des Gelenkdruckes bei Verwendung einer medizintechnischen Fluidpumpe sowie eine Vorrichtung zur Durchführung des Verfahrens.

Bei verschiedenen medizinischen Eingriffen in das Körperinnere werden Fluide, z. B. Gase oder Flüssigkeiten in das Körperinnere ein- und ausgeführt. Beispielshaft genannt sei hier die Arthroskopie, bei der beispielsweise im Rahmen einer Kniegelenksuntersuchung oder einer therapeutischen Behandlung das Knie mit einer Spülflüssigkeit gespült wird. Eine andere beispielhafte Behandlung ist die Laparoskopie, in der während eines therapeutischen Eingriffs Gase (z. B. $CO_2$) in das Körperinnere geführt werden. Im Rahmen dieser Prozeduren ist die Messung, die Regelung und vor allem die Begrenzung des Druckes im Körperinneren von besonderer Bedeutung. Bei therapeutischen Eingriffen ist es insbesondere notwendig, einen gewissen Fluidfluss zu gewährleisten, um beispielsweise Rauch oder Blut aus dem Körperinneren auszuspülen, gleichzeitig aber den Druck zu begrenzen, um das Körpergewebe nicht unnötig zu schädigen. Hierzu sind im Stand der Technik verschiedene Lösungen bekannt. Eine einfache Lösung des Problems besteht darin, einen Drucksensor unmittelbar in das Körperinnere einzuführen. Der Nachteil dieser Lösung besteht unter anderem darin, dass zusätzlicher Platz im Körperinneren benötigt wird, der insbesondere bei kleinen Körperhöhlen (z. B. im Rahmen der Arthroskopie) nicht zur Verfügung steht. Weiterhin vergrößert jede zusätzliche Leitung in das Körperinnere die immer bestehende Infektionsgefahr. Eine andere Lösung besteht darin den Druck in der Zuführleitung zu messen. Dieser Druck weicht jedoch auf Grund der strömungsdynamischen Verhältnisse der Zu- und Abführleitungen mehr oder weniger stark vom Ist-Druck im Körperinneren ab. Da diese Abweichung des gemessenen Druckes vom Ist-Wert in nicht linearer Weise von einer Reihe von Parametern (z. B. Strömungsgeschwindigkeit, Leitungslänge, Leitungsdurchmesser, etc.) abhängt, ist eine einfache Korrektur nicht möglich.

US 2012/0283691 A1 beschreibt ein Infusionssystem zur intravasalen Infusion von Flüssigkeiten mit automatischer Erkennung einer Verstopfung der Infusionsleitung.

US 2015/0085799 A1 beschreibt eine vollautomatische Vorrichtung für medizinische Notfälle, die durch Nicht-Fachleute bedienbar sein soll, inklusive einer elektronisch geregelten Beatmungseinheit.

US 2010/0094113 A1 beschreibt ein automatisches Analysensystem zur Kontrolle von Blutkomponenten (z.B. Glukosegehalt des Blutes) unter gleichzeitiger Überwachung des Blutdruckes.

Vor diesem Hintergrund stellt sich die Aufgabe, eine medizintechnische Vorrichtung zum Einbringen von Fluiden in Körperhöhlen anzugeben, die die vorgenannten Nachteile überwindet. Zur Lösung des Problems wird die Vorrichtung gemäß Anspruch 1 vorgeschlagen. Vorteilhafte Ausgestaltungen sind Gegenstand der auf den Anspruch 1 zurückbezogenen Unteransprüche.

Das offenbarte Verfahren basiert im Wesentlichen darauf, dass zur Messung und Regelung des Körperinnendrucks ein mathematisches Modell nach Art eines Kalman-Filters herangezogen wird. Hierzu wird das Gesamtsystem bestehend aus Druckregler, Pumpenmotor, Zuführleitung, Drucksensor, medizinische Zuführvorrichtung (z.B. Trokar mit Optik), Körperhöhle und Fluidauslass (z.B. Absaugvorrichtung) durch einen Satz von Differentialgleichungen beschrieben und in einem sog. Zustandsraummodell zusammengefasst. Unter der Voraussetzung, dass die Parameter des Modells hinreichend genau bestimmt sind, ergibt sich bei gleicher Eingangsgröße eine Schätzung für den Sensordruck und dem Druck im Körperinneren. Durch einen Vergleich zwischen dem tatsächlichen und dem geschätzten Sensordruck können Abweichungen (sog. Beobachterfehler) detektiert werden. Diese können zum Beispiel durch unterschiedliche Anfangszustände (z.B. liegen zum Beginn der Operation keine a *priori* Informationen über den Körperinnendruck vor) oder durch ein verrauschtes Messsignal des Drucksensors hervorgerufen werden. Wird der Beobachterfehler mit einem Gütekriterium bewertet und das Ergebnis anschließend auf die Energiespeicher des Modells zurückgeführt, so klingt der Fehler ab und es ergibt sich im Ergebnis eine präzise Schätzung für den Druck im Körperinneren. Der Vorteil des vorgeschlagenen Verfahrens besteht unter anderem darin, dass für die Messung des Körperinnendrucks kein zusätzlicher Drucksensor benötigt wird. Im Ergebnis ergibt sich eine deutlich bessere Messung des Körperinnendruckes auch unter Änderung der Einflussparameter, w. z. B. beim Ein- und Ausschalten von Absaugpumpen oder Ähnlichem.

Das offenbarte Verfahren ist wie oben beschrieben Weise so ausgestaltet, dass das Schätzsystem als Kalman Filter realisiert ist. Derartige Kalman Filter sind z.B. in Lehrbüchern der Regelungstechnik erläutert. Sie erlauben eine Berücksichtigung von Gewichtungsmatrizen, die ein Systemrauschen (bedingt durch Parameterschwankungen, Toleranzen, etc.) und/oder ein Messrauschen (Störeinflüsse, Unzulänglichkeiten der Sensorik, etc.) repräsentieren. Dadurch wird die Genauigkeit, mit der das Modell das reale System abbildet, verbessert. Die Art des Kalman-Filters kann dabei kontinuierlich oder zeitdiskret ausgestaltet sein und gilt für lineare und nichtlineare Systeme.

Eine besondere Ausgestaltung einer Vorrichtung, die das oben beschriebene Verfahren realisiert ist eine peristaltische Schlauchpumpe, wie sie beispielsweise in der Arthroskopie verwendet wird. Diese enthält einen regelbaren Motor, welcher die peristaltische Pumpleistung erbringt. Über eine Zuführleitung (z.B. Schlauch und einen Trokar mit Optik) wird die gepumpte Flüssigkeit in das Körperinnere (z. B. das Kniegelenk) geleitet. Aus dem Kniegelenk erfolgt eine Ableitung durch eine zweite Leitung, entweder in Form einer einfachen Drainageleitung oder in Form einer an die Leitung angeschlossenen Absaugpumpe. Durch das offenbarte Verfahren wird der Ist-Druck im Gelenk unter Verwendung der Messdaten aus dem Drucksensor geschätzt und durch den Pumpenmotor geregelt.

Eine alternative Ausführungsform der Erfindung besteht in einem Insufflator, wie er für die Laparoskopie verwendet wird. Durch den Insufflator wird ein Gas in das Körperinnere (z. B. das Abdomen) geleitet. Der Gasabfluss aus dem Körperinneren wird auch hier beispielsweise durch eine Absaugleitung realisiert. Auch hier findet eine Druckmessung in der Zuführleitung statt. Der Ist-Druck im Körperinneren wird durch das erfindungsgemäße Verfahren geschätzt und dient der Regelung des Insufflators. Im Ergebnis wird eine Vorrichtung erhalten, die eine präzise Messung und Regelung des Druckes auch unter extremen Bedingungen, z. B. beim Ein- oder Ausschalten einer Absaugvorrichtung gewährleistet.

Ausführungsbeispiele der Erfindung sind in den Zeichnungen dargestellt und werden nachfolgend näher erläutert.

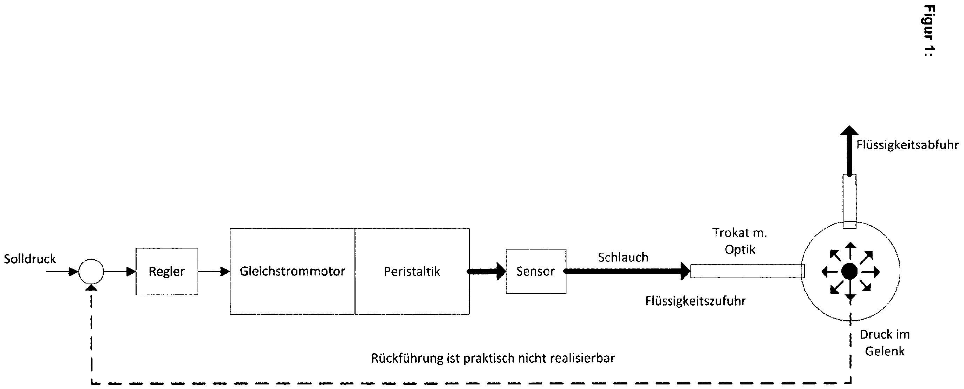

Figur 1 zeigt schematisch die Wunschvorstellung der Therapeuten: Durch eine direkte fehlerfreie Druckmessung im Gelenk wird eine Pumpe geregelt, welche den Flüssigkeitsstrom in das Gelenk gewährleistet. Da eine derartige direkte Druckmessung praktisch nicht möglich ist, beschreibt die Figur 1 nur das angestrebte Ziel.

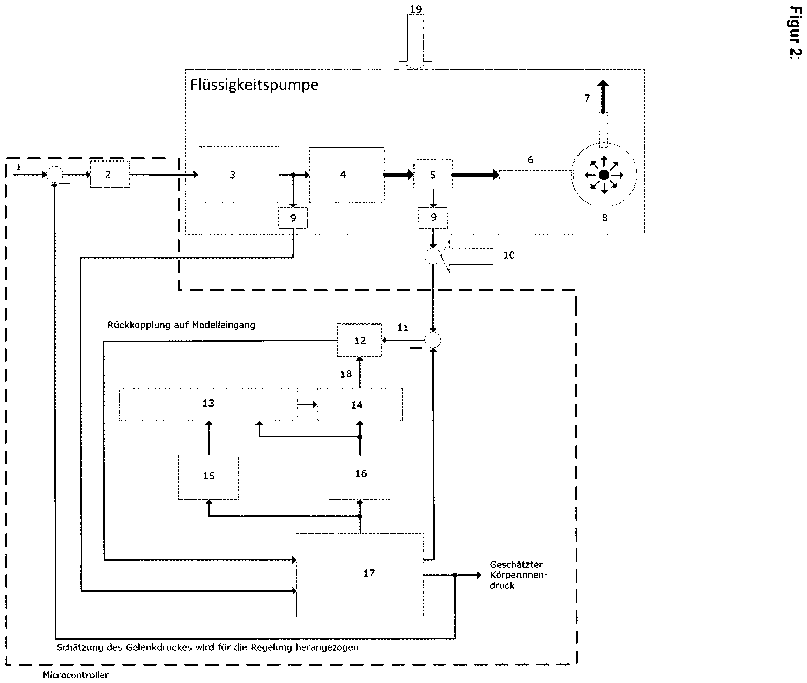

Figur 2 zeigt ein Beispiel einer erfindungsgemäßen Lösung: Der tatsächliche Druck im Gelenk wird basierend auf den Daten des Drucksensors im Schlauch unter Verwendung des Kalman Filters geschätzt. Festgestellte Abweichungen des gemessenen Druckes vom geschätzten Druck werden über die sog. Kalman Gains in das mathematische Modell eingearbeitet und für die weitere Schätzung herangezogen. Des Weiteren zeigt Figur 2 die für dieses Modell herangezogen Zustandsgleichungen.

Die in der Figur gezeigten Ziffern haben folgende Bedeutung:

| 1 | Solldruck |
|---|---|
| 2 | Regler |
| 3 | Gleichstrommotor |
| 4 | Peristaltik |
| 5 | Sensor |
| 6 | Trokar mit Optik |
| 7 | Flüssigkeitsabfuhr |
| 8 | Körperinnendruck |
| 9 | A/D |
| 10 | Messrauschen |
| 11 | Beobachterfehler |
| 12 | $\underline{L}$ |
| 13 | $\underline{\dot{P}} = \underline{AP} + \underline{PA}^T + \underline{Q} - \underline{PC}^T \underline{S}^{-1} \underline{CP}$ |
| 14 | $\underline{L} = PC^T S^{-1}$ |
| 15 | $\underline{A} = \dfrac{\partial f}{\partial x}\bigg|_{\tilde{x}}$ |
| 16 | $C = \dfrac{\partial g}{\partial x}\bigg|_{\tilde{x}}$ |

(fortgesetzt)

| 17 | $\dot{\tilde{x}} = f(\tilde{x}, u) + \vartheta$ <br> $\tilde{y} = g(\tilde{x})$ <br> Modell der Flüssigkeitspumpe |
|---|---|
| 18 | *Kalman-Gains* |
| 19 | Systemrauschen |

Die hierin verwendeten Variablen haben die folgende Bedeutung:

| $\underline{L}$ | *Kalman-Gains* |
|---|---|
| $\underline{P}$ | Kovarianz-Matrix, welche als Lösung aus $\underline{\dot{P}}$ resultiert |
| $\underline{\dot{P}}$ | nichtlineare Matrix-Ricatti-Differentialgleichung zur Bestimmung von $\underline{P}$ |
| $\underline{Q}$ | Prozessrausch-Matrix |
| $\underline{S}$ | Messrausch-Matrix |
| $\underline{A}$ | Systemmatrix (Jacobi-Matrix) |
| $\underline{C}$ | Beobachtungsmatrix |
| $\dot{\tilde{x}}$ | nach der Zeit differenzierter Zustandsvektor |
| $f(\tilde{x}, u)$ | Satz von rechtseitigen Funktionen der Differentialgleichungen erster Ordnung zur Beschreibung des dynamischen und statischen Verhaltens der Flüssigkeitspumpe oder des Insufflators |
| $\vartheta$ | mittelwertfreies, normalverteiltes Prozessrauschen |
| $\tilde{y}$ | Ausgangsgröße |
| $g(\tilde{x})$ | Ausgabefunktion |

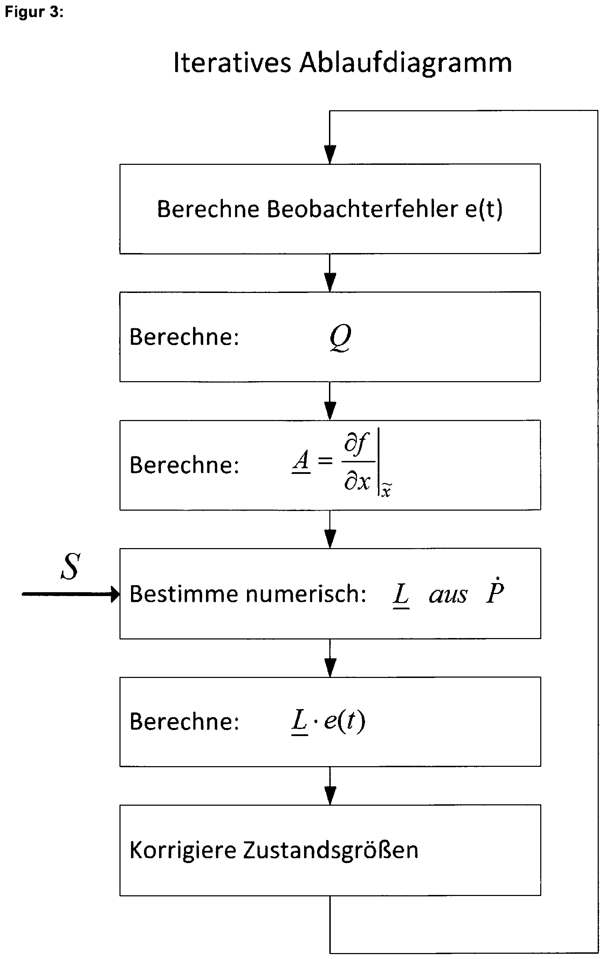

Die praktische Durchführung des oben genannten Verfahrens erfolgt zweckmäßigerweise auf einem Mikrocontroller, der Bestandteil der medizintechnischen Vorrichtung ist. Diese ist in üblicher Weise mit Ein- und Ausgängen sowie Speichern versehen. Die mathematischen Operationen werden in Form eines Softwaremoduls abgearbeitet. Ein Ablaufdiagramm des Softwaremoduls ist in der Figur 3 dargestellt. Die Software kann auf einem eigenen Speicherchip, zum Beispiel einem EPROM hinterlegt sein.

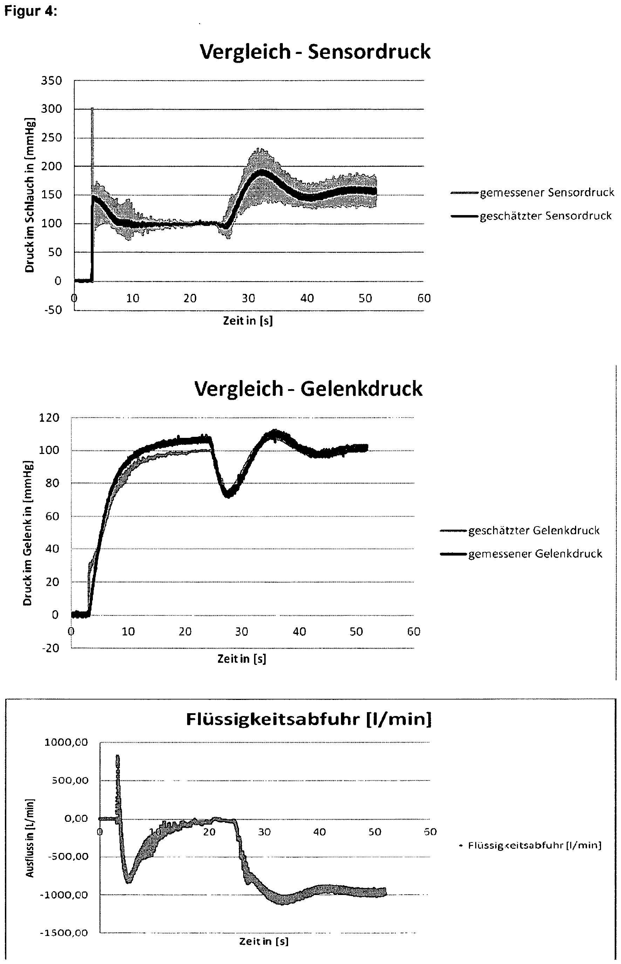

Figur 4 zeigt die erhaltenen Ergebnisse. In der oberen Figur wird der gemessene mit dem geschätzten Sensordruck und in der mittleren Figur der gemessene mit dem geschätzten Gelenkdruck verglichen. Die in Figur 4 dargestellten Verläufe zeigen einen Regelvorgang, wobei als Regelgröße der vom Kalman Filter rekonstruierte Gelenkdruck verwendet wird. Anhand der mittleren Figur wird deutlich, dass der geschätzte Gelenkdruck dem gemessenem nahezu identisch ist. In der unteren Figur ist die vom Kalman-Filter geschätzte Flüssigkeitsabfuhr dargestellt.

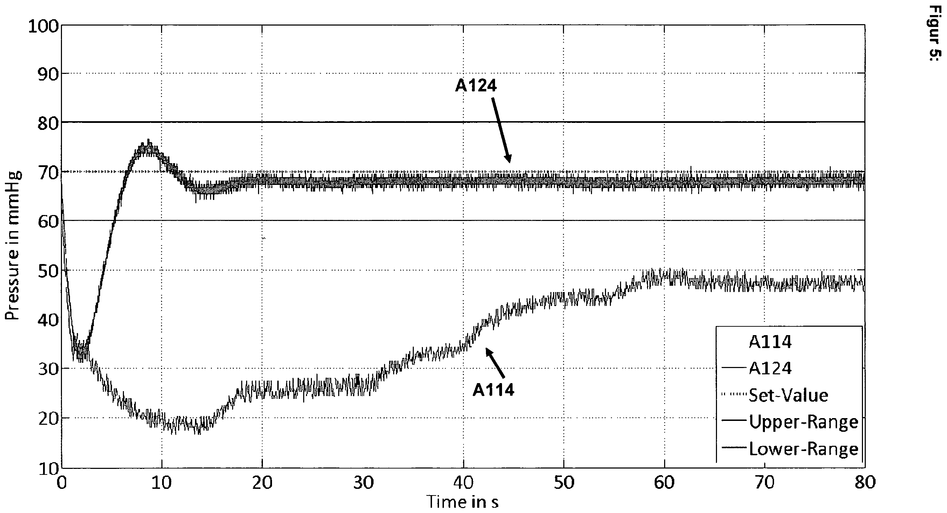

Figur 5 zeigt Ergebnis von Vergleichsmessungen überreicht. In einem Experiment wurden zwei medizinische Pumpen verglichen. Dabei handelt es sich einmal um eine klassische peristaltische Pumpe mit einem Drucksensor in der Zuführleitung (gekennzeichnet als A114) und eine erfindungsgemäße Pumpe mit einem mathematischen Schätzsystem, welche als Eingangsvariable den in der Zuführleitung gemessenen Druck eines Drucksensors erhält (gekennzeichnet als A124). Beide Pumpen wurden an einen Dummy angeschlossen, welcher die Verhältnisse in einer tatsächlichen Körperhöhle simuliert. Dargestellt sind die innerhalb des Dummys durch einen separaten Sensor tatsächlich gemessenen Werte. Dabei wurde an beiden Pumpen ein Solldruck von 70 mmHg eingestellt (gekennzeichnet als "set value"), wobei Druckschwankungen von $\pm$ 10 mmHg als akzeptabel angesehen wurden (gekennzeichnet als "upper ränge", bzw "lower ränge").

Der Vergleich der Kurven zeigt, dass die erfindungsgemäß ausgestattete Pumpe bereits nach fünf Sekunden einen Druck innerhalb des erwähnten Korridors von 60 bis 80 mmHg erreicht und nach einem kurzen Einschwingen den angestrebten Druck äußerst präzise einhält. Demgegenüber wird bei der aus dem Stand der Technik bekannten Pumpe erst nach 60 Sekunden ein halbwegs stabiler Wert erzielt, der allerdings außerhalb des eingestellten Bereiches liegt. Selbst wenn hier eine Nachkalibrierung vorgenommen würde, blieben die deutlichen Abweichungen vom Sollwert innerhalb der ersten 60 Sekunden sowie die größeren Schwankungen im zeitlichen Verlauf erhalten.

Der Vergleich der beiden Pumpen zeigt daher deutlich den überraschenden Vorteil des erfindungsgemäßen Systems, welcher darin besteht, dass der Drucksollwert deutlich schneller erreicht wird und auch deutlich besser über die Zeit konstant gehalten wird. Eine derartige Verbesserung kann dem Stand der Technik nicht entnommen werden.

Der Fachmann auf dem Gebiet kann, basierend auf der vorliegenden Beschreibung, insbesondere der Beschreibungen in der Figur 2 und der zum Zeitpunkt der Anmeldung hinlänglich bekannten Fachliteratur weitere Ausführungsformen der Erfindung realisieren, ohne weiter erfinderisch tätig werden zu müssen.

**Patentansprüche**

1.  Medizintechnische Vorrichtung zum Einbringen von Fluiden in Körperhöhlen, enthaltend
    eine regelbare Fluidpumpe,
    eine Zuführleitung zur Einleitung eines Fluides in eine Körperhöhle,
    einen Drucksensor in der Zuführleitung,
    eine zweite Leitung zum Ableiten des Fluides aus der Körperhöhle, mindestens einen Mikroprozessor, mindestens einen Speicher und mindestens eine Software,
    wobei der Drucksensor den Druck in der Zuführleitung misst und der Software als Eingangsvariable zur Verfügung stellt,
    **dadurch gekennzeichnet, dass** die Software ein mathematisches Schätzsystem enthält, welches mathematisch einen Zustandsraum beschreibt, welcher den tatsächlichen Druck in der Körperhöhle abschätzt und mittels dieses Schätzwertes die Pumpleistung der Fluidpumpe regelt,
    wobei das in der Software enthaltene, mathematische Schätzsystem nach Art eines Kalman-Filters ausgestaltet ist.

2.  Medizintechnische Vorrichtung zum Einbringen von Fluiden in Körperhöhlen gemäß Anspruch 1, **dadurch kennzeichnet, dass** es sich bei dem Fluid um ein Gas handelt.

3.  Medizintechnische Vorrichtung zum Einbringen von Fluiden in Körperhöhlen gemäß Anspruch 1, **dadurch kennzeichnet, dass** es sich bei dem Fluid um eine Flüssigkeit handelt.

**Claims**

1.  A medical device for introducing fluids into body cavities, including
    a controllable fluid pump,
    a feed line for introduction of a fluid into a body cavity,
    a pressure sensor in the feed line,
    a second line for discharging the fluid from the body cavity,
    at least one microprocessor, at least one memory, and at least one software,
    the pressure sensor measuring the pressure in the feed line and providing it to the software as an input variable,
    **characterized by** that the software describes a mathematical estimation system mathematically describing a state space that estimates the actual pressure in the body cavity and controls, by means of this estimated value, the pumping power of the fluid pump,
    wherein the mathematical estimation system included in the software is configured in the manner of a Kalman filter.

2.  The medical device for introducing fluids into body cavities according to claim 1, **characterized by** that the fluid is a gas.

3.  The medical device for introducing fluids into body cavities according to claim 1, **characterized by** that the fluid is a liquid.

**Revendications**

1.  Dispositif médical pour l'introduction de fluides dans des cavités corporelles, comportant
    une pompe à fluides commandable,
    une conduite d'alimentation pour l'introduction d'un fluide dans une cavité corporelle,

un capteur de pression dans la conduite d'alimentation,

une deuxième conduite pour l'évacuation du fluide de la cavité corporelle,

au moins un microprocesseur, au moins une mémoire, et au moins un logiciel,

le capteur de pression mesurant la pression dans la conduite d'alimentation et la mettant à la disposition du logiciel comme variable d'entrée,

**caractérisé en ce que** le logiciel décrit un système d'estimation mathématique, qui décrit mathématiquement un espace d'état estimant la pression actuelle dans la cavité corporelle et commande, au moyen de cette valeur estimée, la puissance de pompage de la pompe à fluides,

dans lequel le système d'estimation mathématique compris dans le logiciel est configuré de façon d'un filtre de Kalman.

2. Dispositif médical pour l'introduction de fluides dans des cavités corporelles selon la revendication 1, **caractérisé en ce que** le fluide est un gaz.

3. Dispositif médical pour l'introduction de fluides dans des cavités corporelles selon la revendication 1, **caractérisé en ce que** le fluide est un liquide.

**Figur 1:**

Flüssigkeitsabfuhr

Druck im Gelenk

Trokat m. Optik

Flüssigkeitszufuhr

Schlauch

Sensor

Rückführung ist praktisch nicht realisierbar

Peristaltik

Gleichstrommotor

Regler

Solldruck

7

**Figur 2**:

Flüssigkeitspumpe

Rückkopplung auf Modelleingang

Schätzung des Gelenkdruckes wird für die Regelung herangezogen

Microcontroller

Geschätzter Körperinnen-druck

**Figur 3:**

# Iteratives Ablaufdiagramm

Berechne Beobachterfehler e(t)

Berechne: $Q$

Berechne: $\underline{A} = \left.\dfrac{\partial f}{\partial x}\right|_{\tilde{x}}$

$S$

Bestimme numerisch: $\underline{L}$ aus $\dot{P}$

Berechne: $\underline{L} \cdot e(t)$

Korrigiere Zustandsgrößen

**Figur 4:**

## Vergleich - Sensordruck

gemessener Sensordruck
geschätzter Sensordruck

## Vergleich - Gelenkdruck

geschätzter Gelenkdruck
gemessener Gelenkdruck

## Flüssigkeitsabfuhr [l/min]

• Flüssigkeitsabfuhr [l/min]

**Figur 5:**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120283691 A1
- US 20150085799 A1

- US 20100094113 A1